# FASCICULE DE BREVET EUROPEEN

(11) **EP 3 793 675 B1**
(45) Date de publication et mention de la délivrance du brevet: **18.12.2024**
(21) Numéro de dépôt: 19730205.2
(22) Date de dépôt: 15.05.2019
(51) Int. Cl.: A61N 2/12, A61N 2/02

(54) **APPAREIL POUR GENERER UN CHAMP MAGNETIQUE PULSE A TRES BASSE FREQUENCE PORTE PAR UN CHAMP MAGNETIQUE ALTERNATIF A TRES BASSE FREQUENCE**
VORRICHTUNG ZUR ERZEUGUNG EINES SEHR NIEDERFREQUENTEN GEPULSTEN MAGNETFELDES, DAS VON EINEM SEHR NIEDERFREQUENTEN WECHSELMAGNETFELD GETRAGEN WIRD
DEVICE FOR GENERATING A VERY-LOW-FREQUENCY PULSED MAGNETIC FIELD CARRIED BY A VERY-LOW-FREQUENCY ALTERNATING MAGNETIC FIELD

(30) Priorité: 18.05.2018 FR 1854157
(43) Date de publication de la demande: 24.03.2021
(73) Titulaire: G.C. Technology, 13854 Aix-en-Provence (FR)
(72) Inventeur: CREPIN, Gérard, 13090 AIX EN PROVENCE (FR); RUDENT, Pascal, 13390 AURIOL (FR)
(74) Mandataire: Cabinet Beau de Loménie
(86) Numéro de dépôt international: PCT/FR2019/051109
(87) Numéro de publication internationale: WO 2019/220060

(56) Documents cités:
- EP-A2- 1 364 679
- DE-U1- 202017 003 178
- US-A- 5 935 054
- US-A1- 2018 078 780

## Description

### Arrière-plan de l'invention

La présente invention se rapporte au domaine général de la magnétothérapie, et notamment aux appareils qui génèrent un courant électrique induit sinusoïdal à basse fréquence destiné à être appliqué sur une zone du corps humain dans le but d'exercer une action antalgique et anti-inflammatoire.

De façon connue, pour générer un courant électrique induit, la magnétothérapie utilise un champ magnétique variable notamment pour soulager les douleurs articulaires et les douleurs péri-articulaires, ce champ magnétique variable étant créé par une source magnétique, une bobine ou un aimant. Dans le cas d'aimants, ces derniers sont mis en mouvement pour créer le courant induit.

Les champs magnétiques ont également un impact sur la microcirculation qui régule l'apport sanguin au niveau de la périphérie de l'organisme (membres) mais aussi au niveau des différents organes. Comme le montre une étude de 2014 par Funk, l'exposition pendant 5 minutes à un champ magnétique sinusoïdal de 10 à 15mT variant entre des fréquences de 4 à 12Hz augmente la microcirculation au niveau de la main chez des sujets sains. Dans une population de jeunes diabétiques de type 1 qui ont un déficit microcirculatoire caractéristique de leur pathologie, une étude de 2008 de Nikolaeva et al montre aussi une forte augmentation de la microcirculation cutanée au niveau de la jambe après exposition pendant 15 minutes à un champ magnétique sinusoïdal de 45mT à 16Hz par rapport à un groupe exposé à un appareil placébo.

La magnétothérapie peut être mise en oeuvre à l'aide d'un appareil (le plus souvent portatif) déplacé au niveau de la zone du corps à traiter, cet appareil renfermant des aimants, par exemple rotatifs, et un moteur pour permettre à ces aimants de générer un champ magnétique variable induisant un champ électrique proportionnel à la vitesse de la variation (loi de Faraday).

On connaît ainsi du document WO 2008/014902 un appareil portatif pour générer un champ magnétique sinusoïdal à des fins thérapeutiques, cet appareil comprenant quatre secteurs angulaires d'aimant qui sont plats, de même forme géométrique, centrés sur un même axe de rotation et espacés angulairement les uns des autres, la polarité de deux secteurs angulaires d'aimant adjacents étant opposée. Ainsi disposés, les secteurs angulaires d'aimant sont entraînés en rotation par un moteur afin de générer un champ magnétique sinusoïdal à une fréquence prédéfinie.

Il est par ailleurs connu que l'effet thérapeutique obtenu est lié aux courants électriques induits par la rotation des secteurs angulaires d'aimant, à condition que l'intensité de ces courants dépasse un certain seuil. Il est également connu que les courants induits sont proportionnels à la vitesse de déplacement linéaire des aimants. Or, pour une vitesse de rotation donnée, la vitesse de déplacement linéaire est plus importante à l'extrémité des secteurs angulaires d'aimant qu'au niveau de leur axe de rotation. De plus, pour obtenir un champ magnétique sinusoïdal avec l'appareil décrit dans le document WO 2008/014902, il est nécessaire d'utiliser des aimants en forme de secteurs angulaires, ce qui diminue davantage le champ magnétique au centre de l'appareil.

Aussi, l'appareil décrit dans le document WO 2008/014902 présente l'inconvénient que l'intensité des courants électriques induits au niveau du centre de l'appareil ne dépasse pas le seuil nécessaire à l'obtention d'un effet thérapeutique. Or, le centre de l'appareil est typiquement la zone la plus sollicitée par l'utilisateur de l'appareil. En effet, de manière naturelle, l'utilisateur a tendance à centrer l'appareil sur la douleur ou la pathologie à traiter.

Un autre inconvénient de l'appareil décrit dans ce document est que l'espace entre les aimants génère un signal en double pic dans une zone proche de la surface de l'appareil, et donc un taux important de distorsion harmonique.

Par ailleurs, les effets thérapeutiques d'un tel appareil étant obtenus pour des fréquences très basses, de préférence inférieure à 10 Hz, il n'est pas souhaitable d'augmenter le champ *V̅ X B̅* en augmentant la vitesse de rotation des aimants. En effet, cette augmentation du champ *V̅ X B̅* aurait pour conséquence de réduire les effets thérapeutiques obtenus.

Par ailleurs, il est fait référence aux documents US2018/0078780 A1 et DE202017003178 U1.

### Objet et résumé de l'invention

La présente invention a donc pour but de proposer un appareil destiné à la magnétothérapie qui ne présente pas les inconvénients précités.

Conformément à l'invention, ce but est atteint grâce à un appareil selon la revendication 1.

La bobine de fil conducteur centrée sur l'axe de rotation des secteurs angulaires d'aimant et alimentée en courant électrique pulsé crée un courant électrique induit fort au centre de la zone d'action de l'appareil, ce qui permet de compenser la non homogénéité du courant induit par les secteurs angulaires d'aimant en rotation. De même, la bobine de fil conducteur a peu d'effet en périphérie des secteurs angulaires d'aimant (au contraire de ces derniers), de sorte que l'association de ces deux éléments permet d'obtenir un courant électrique induit fort en tout point de la zone d'action de l'appareil. Ainsi, l'intensité du courant électrique induit est et au-dessus d'un seuil prédéfini entre l'axe de rotation et l'extrémité libre des secteurs angulaires d'aimant.

Par ailleurs, les inventeurs ont constaté que la génération d'un champ magnétique pulsé à basse fréquence porté par un champ magnétique alternatif augmente la microcirculation sanguine de la zone du corps humain sur laquelle est appliqué l'appareil. En particulier, des mesures montrent de façon inattendue une augmentation de la microcirculation sanguine significativement plus importante qu'avec la seule mise en rotation des secteurs angulaires d'aimant ou avec la seule alimentation de la bobine de fil conducteur. Le résultat de ces mesures mettent en évidence que l'effet en termes de microcirculation sanguine est supérieur à la somme des effets de chacun des deux champs.

De la sorte, la génération par l'appareil selon l'invention d'une variation brutale du champ magnétique en alimentant la bobine de fil conducteur par un courant électrique pulsé permet de créer une tension induite dont les effets antalgiques et anti-inflammatoires sont importants.

De préférence, la bobine de fil conducteur est alimentée par un courant électrique pulsé se présentant sous la forme d'impulsions régulières ayant une durée de 1ms et une fréquence de 20 Hz et générant une intensité permettant de générer un champ magnétique de 1 à 2 mT. La bobine de fil conducteur de diamètre très faible permet d'obtenir un courant induit important malgré le faible courant magnétique avec une faible consommation d'énergie. Le système ainsi constitué modifie très peu la valeur maximale du champ magnétique et peut être portable et autonome.

De préférence également, l'appareil comprend en outre des moyens pour faire varier pendant un cycle d'utilisation la fréquence des impulsions du courant électrique pulsé. Cette caractéristique permet de modifier le spectre fréquentiel afin de répondre à différentes pathologies par un signal plus adapté.

De préférence encore, l'appareil comprend en outre des moyens pour faire varier pendant un cycle d'utilisation la fréquence de rotation des paires de secteurs d'aimant. Ainsi le signal sinusoïdal généré passe par différentes fréquences dont chacune a montré son efficacité.

La fréquence de rotation des paires de secteurs d'aimant est avantageusement inférieure ou égale à 10 Hz.

La bobine de fil conducteur peut être une bobine circulaire centrée sur l'axe de rotation des secteurs angulaires d'aimant. La bobine de fil conducteur peut être une bobine à double anneaux qui permet de maximiser l'effet à un endroit précis (bobines dites « papillon »).

De préférence encore, chaque secteur angulaire d'aimant comprend une même forme géométrique avec une ouverture angulaire intérieure au niveau de l'axe de rotation de 90°, une ouverture angulaire extérieure au niveau d'une extrémité libre opposée à l'axe de rotation comprise entre 20° et 50°, et deux bords latéraux définissant un rayon s'étendant sur une distance qui est comprise entre un tiers et deux-tiers d'une distance séparant l'axe de rotation de l'extrémité libre des secteurs angulaires d'aimant. Cette forme particulière des secteurs angulaires d'aimant permet d'obtenir un champ magnétique sinusoïdal dont l'intensité est homogène et au-dessus d'un seuil prédéfini entre l'axe de rotation et l'extrémité libre des secteurs angulaires d'aimant.

L'appareil peut comprendre en outre une couche de circuit magnétique disposée au dos des paires de secteurs angulaires d'aimants. L'appareil peut également comprendre en outre une couche de blindage magnétique disposée autour des paires de secteurs angulaires d'aimant.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- la figure 1 est une vue schématique d'un appareil selon un mode de réalisation de l'invention ;
- la figure 2 est une vue en coupe selon II-II de la figure 1 ;
- les figures 3A et 3B sont des exemples de champs magnétiques pulsés générés par l'appareil selon l'invention ; et
- la figure 4 représente des courbes illustratives de la microcirculation sanguine obtenues par l'appareil selon l'invention, par un appareil à aimants tournant, et par un appareil à bobine de fil conducteur.

### Description détaillée de l'invention

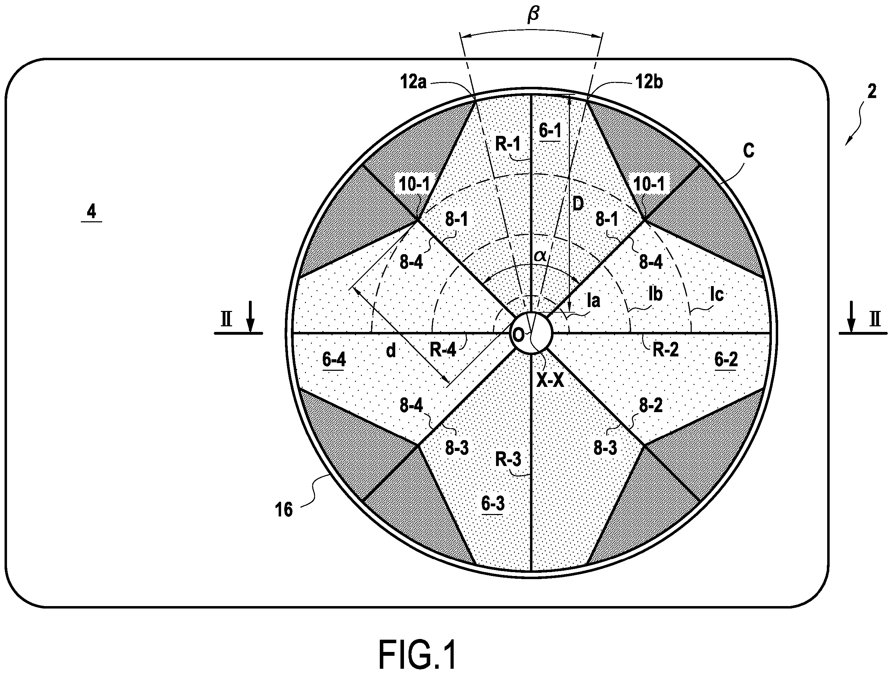

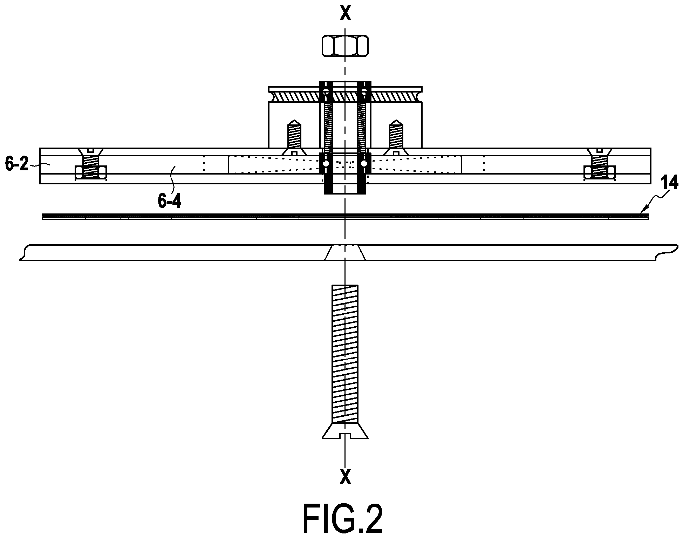

Les figures 1 et 2 représentent de façon schématique un appareil portatif 2 selon l'invention pour générer un champ magnétique pulsé à basse fréquence porté par un champ magnétique alternatif et destiné à être appliqué sur une zone du corps humain.

Cet appareil 2 comprend un boîtier 4 à l'intérieur duquel sont assemblés deux paires de secteurs angulaires d'aimant 6-1 à 6-4 comme décrit dans la demande de brevet FR 17 60443 déposée par la Demanderesse.

Comme décrit dans cette demande de brevet FR 17 60443, les secteurs angulaires d'aimant 6-1 à 6-4 ont une même forme géométrique, sont centrés sur un même axe de rotation X-X, et sont inscrits à l'intérieur d'un cercle C de diamètre D et de centre O.

De façon plus précise, les secteurs angulaires d'aimant 6-1 à 6-4 sont espacés angulairement les uns des autres autour de l'axe de rotation X-X et sont disposés de façon à ce que la polarité (Nord ou Sud) de deux secteurs angulaires d'aimant adjacents soit opposée.

L'appareil 2 comprend en outre des moyens pour mettre en rotation les paires de secteurs angulaires d'aimant autour de l'axe de rotation X-X. Dans un exemple de réalisation, ces moyens se présentent sous la forme d'un moteur électrique et d'une transmission par courroie. Bien entendu, tout autre moyen pourrait être envisagé pour assurer cette mise en rotation.

De préférence, les quatre secteurs angulaires d'aimant 6-1 à 6-4 présentent chacun une forme symétrique par rapport à un rayon de symétrie R-1 à R-4, respectivement, du cercle C à l'intérieur duquel ils sont inscrits.

Chaque secteur angulaire d'aimant 6-1 à 6-4 comprend deux bords latéraux 8-1 à 8-4 qui sont symétriques par rapport au rayon de symétrie du secteur angulaire d'aimant et qui sont en regard de ou en contact direct avec des bords latéraux correspondants de deux secteurs angulaires d'aimant adjacents.

De plus, les deux bords latéraux 8-1 à 8-4 de chaque secteur angulaire d'aimant définissent un rayon de jointure (délimité entre le centre O du cercle C et le point 10-1 à 10-4 du bord latéral le plus éloigné du centre O) qui s'étend sur une distance d correspondant à deux-tiers du rayon D/2 du cercle C (c'est-à-dire à deux-tiers de la distance séparant l'axe de rotation X-X de l'extrémité libre des secteurs angulaires d'aimant). Pour des raisons de commodité, seuls les points 10-1 du secteur angulaire d'aimant 6-1 sont représentés sur la figure 1.

En outre, les bords latéraux 8-1 à 8-4 de chaque secteur angulaire d'aimant forme entre eux un angle α de 90° (on dit également que l'ouverture angulaire intérieure α de chaque secteur angulaire d'aimant au niveau de l'axe de rotation X-X est de 90°).

De préférence également, chaque secteur angulaire d'aimant comprend en outre une ouverture angulaire extérieure β au niveau d'une extrémité libre opposée à l'axe de rotation X-X qui est comprise entre 20° et 50°, et de préférence égale à 45°.

En d'autres termes, l'extrémité libre de chaque secteur angulaire d'aimant 6-1 à 6-4 est de préférence délimitée entre deux points 12a, 12b situés sur le cercle C dans lequel sont inscrits les secteurs angulaires d'aimant. Ces points sont symétriques et les rayons formés par les points O et 12a, d'une part, et O et 12b, d'autre part, forment entre eux un angle β compris entre 20° et 50°, et de préférence égal à 45°.

Les secteurs angulaires d'aimant 6-1 à 6-4 de l'appareil selon l'invention sont mis en rotation autour de l'axe de rotation X-X à une vitesse de préférence de 300 tours/minute, ce qui génère un courant électrique induit sinusoïdal à une fréquence de préférence inférieure ou égale à 10Hz. Cette mise en rotation des secteurs angulaires d'aimant 6-1 à 6-4 de l'appareil génère un champ magnétique alternatif.

Comme représenté sur la figure 2, l'appareil 2 selon l'invention comprend en outre au moins une bobine de fil conducteur 14 qui est centrée sur l'axe de rotation X-X des secteurs angulaires d'aimant et qui est alimentée en courant électrique pulsé.

Cette bobine 14 est par exemple une bobine circulaire centrée sur l'axe de rotation des secteurs angulaires d'aimant. Elle peut être réalisée en fil de cuivre de diamètre inférieur à 0,5 mm, en une seule couche de spires, son diamètre extérieur couvrant le diamètre extérieur des aimants.

Par ailleurs, cette bobine est alimentée en tension électrique pulsée. On entend par « tension électrique pulsée » une variation de tension électrique qui se présente sous forme d'impulsions régulières et uniformes au cours du temps, dont on peut déterminer les durées de maintien (ou de génération), ainsi que les durées de récurrence (ou fréquence). Il peut également s'agir d'impulsions irrégulières, mais que l'on connaît et que l'on sait reproduire.

De préférence, la tension électrique pulsée alimentant la bobine de fil conducteur 14 se présente sous la forme d'impulsions régulières ayant une durée inférieur à 1 ms, une fréquence de 20 Hz et générant une intensité suffisante pour générer un champ magnétique maximum de 1 à 2 mT.

De la sorte, la bobine de fil conducteur 14 génère un champ magnétique pulsé qui se superpose au champ magnétique alternatif généré par la mise en rotation des secteurs angulaires d'aimant 6-1 à 6-4.

On notera que la bobine de fil conducteur peut être une bobine à double anneaux (bobine type « papillon ») centrée sur l'axe de rotation X-X des secteurs angulaires d'aimant.

On notera également que l'appareil selon l'invention peut comprendre en outre des moyens (de type microcontrôleur) pour faire varier pendant un cycle d'utilisation la fréquence des impulsions du courant électrique pulsé alimentant la bobine de fil conducteur 14 et/ou la fréquence de rotation des paires de secteurs d'aimant 6-1 à 6-4.

Cette caractéristique est particulièrement avantageuse pour faire varier le champ magnétique généré par l'appareil au cours d'un cycle d'utilisation.

On notera encore que les secteurs angulaires d'aimant 6-1 à 6-4 sont entraînés en rotation autour de l'axe de rotation X-X par un moteur électrique (non représenté) indépendant de l'alimentation électrique de la bobine 14. Par ailleurs, pendant l'alimentation de la bobine 14, la polarité de cette dernière reste inchangée. En particulier, la bobine ne subit aucune inversion de sa polarité au cours du fonctionnement de l'appareil selon l'invention.

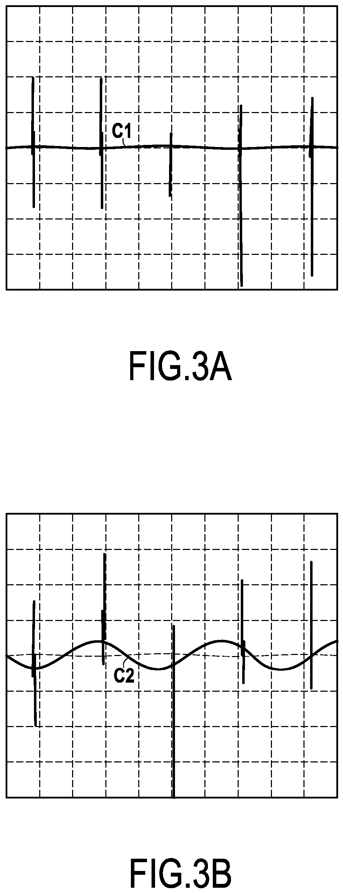

Les figures 3A et 3B représentent des exemples d'un champ magnétique (en fonction du temps) généré par la superposition de ces deux champs.

En particulier, la figure 3A représente un exemple de courbe de courant induit C1 généré par l'appareil selon l'invention au centre de celui-ci, c'est-à-dire au niveau de l'axe de rotation X-X des secteurs angulaires d'aimant. Quant à la figure 3B, elle montre un exemple de courbe de courant induit C2 alternatif à une distance d'un centimètre du centre de l'appareil.

Ces deux courbes de courant induit C1, C2 montrent bien chacune des pulsations portées par une sinusoïde. En particulier, on constate que l'intensité des pulsations portées par la sinusoïde est plus importante au centre de l'appareil et diminue en s'écartant du centre. Ainsi, la bobine de fil conducteur lorsqu'elle est alimentée en courant électrique pulsé crée un courant induit fort au centre de l'appareil et moindre à sa périphérie.

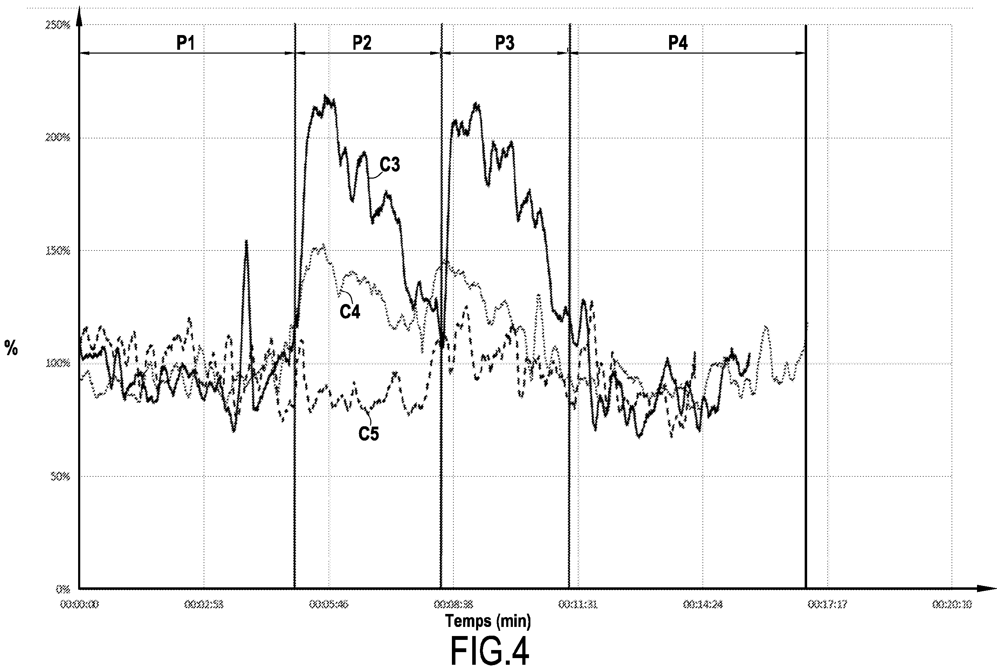

La figure 4 illustre un exemple de test de l'appareil selon l'invention et les résultats obtenus sur la microcirculation sanguine d'une zone du corps humain exposé à l'appareil au cours de plusieurs cycles d'application.

Plus précisément, le test de l'appareil s'est déroulé sur la main d'une personne selon le mode opératoire suivant : une période de repos P1 (d'une durée de 5mn), un premier cycle d'application de l'appareil P2 (d'une durée de 3 mn), un second cycle d'application de l'appareil P3 (d'une durée de 3 mn), et une période de retour à la normale P4 (d'une durée de 15 mn).

A partir d'un imageur de perfusion sanguine, il a été possible de mesurer directement le niveau de perfusion cutanée (c'est-à-dire la microcirculation sanguine) au cours du test. Les données issues de l'imageur ont été normalisées par rapport à la moyenne des valeurs de la dernière minute de la période de repos P1 afin d'exprimer le pourcentage d'augmentation de la microcirculation au cours du test.

La figure 4 montre trois courbes différentes correspondant à trois tests différents : une courbe C3 correspondant à un test au moyen d'un appareil selon l'invention, une courbe C4 correspondant à un test au moyen d'un appareil de magnétothérapie muni d'une bobine de fil conducteur mais dépourvu d'aimants tournant, et une courbe C5 correspondant à un test au moyen d'un appareil muni d'aimants tournant mais dépourvu de bobine de fil conducteur.

On constate que l'effet sur la microcirculation sanguine obtenu par application de l'appareil selon l'invention au cours des cycles d'application P2, P3 (courbe C3) est largement supérieur à la simple addition des effets obtenus par les deux autres appareils (courbes C4 et C5). En effet, on constate une augmentation de 100% de la perfusion sanguine globale sur la main pour le début des deux cycles d'application P2, P3, alors que cette augmentation n'est que de 50% pour l'appareil dépourvu de bobine de fil conducteur (courbe C5). Quant à l'appareil dépourvu d'aimants tournant (courbe C4), il a très peu d'effet sur la microcirculation.

Par ailleurs, on notera que, comme pour l'appareil décrit dans la demande de brevet FR 17 60443, l'appareil selon l'invention peut avoir des secteurs angulaires d'aimant d'épaisseurs variables et être muni d'une couche de circuit magnétique disposée au dos des paires de secteurs angulaires d'aimants et d'une couche de blindage magnétique disposée autour des paires de secteurs angulaires d'aimant (non représentées sur les figures). Dans ce cas, l'antenne sera positionnée côté opposé au circuit magnétique pour que le champ magnétique de l'antenne circulaire ne soit pas atténué par le circuit magnétique.

Par ailleurs, on notera que, comme pour l'appareil décrit dans la demande de brevet FR 17 60443, l'appareil selon l'invention peut avoir des secteurs angulaires d'aimant d'épaisseurs variables et être muni d'une couche de circuit magnétique disposée au dos des paires de secteurs angulaires d'aimants et d'une couche de blindage magnétique disposée autour des paires de secteurs angulaires d'aimant (non représentées sur les figures). Dans ce cas, l'antenne sera positionnée côté opposé au circuit magnétique pour que le champ magnétique de l'antenne circulaire ne soit pas atténué par le circuit magnétique.

## Revendications

1. Appareil (2) pour générer un champ magnétique pulsé à très basse fréquence porté par un champ magnétique alternatif à très basse fréquence et destiné à être appliqué sur une zone du corps humain, l'appareil comprenant :
une pluralité de paires de secteurs angulaires d'aimant (6-1 à 6-4) de polarité inversée, les paires de secteurs angulaires d'aimant étant centrées sur un même axe de rotation (X-X), espacées angulairement les unes des autres, et mises en rotation à une vitesse prédéterminée autour de l'axe de rotation de façon à générer un champ magnétique alternatif à une fréquence prédéfinie ;
au moins une bobine de fil conducteur (14) centrée sur l'axe de rotation des secteurs angulaires d'aimant et alimentée en courant électrique pulsé de façon à générer un champ magnétique pulsé se superposant au champ magnétique alternatif généré par la mise en rotation des secteurs angulaires d'aimant ; et
un moteur électrique indépendant de l'alimentation électrique de la bobine, les secteurs angulaires d'aimant (6-1 à 6-4) étant entraînés en rotation autour de l'axe de rotation (X-X) par ledit moteur électrique.

2. Appareil selon la revendication 1, dans lequel la bobine de fil conducteur (14) est alimentée par une tension électrique pulsée se présentant sous la forme d'impulsions régulières ayant une durée inférieure à 1 ms, une fréquence de 20 Hz et générant une intensité de suffisante pour générer un champ magnétique de 1 à 2 mT.

3. Appareil selon la revendication 2, comprenant en outre des moyens pour faire varier pendant un cycle d'utilisation la fréquence des impulsions du courant électrique pulsé.

4. Appareil selon l'une quelconque des revendications 1 à 3, comprenant en outre des moyens pour faire varier pendant un cycle d'utilisation la fréquence de rotation des paires de secteurs d'aimant.

5. Appareil selon l'une quelconque des revendications 1 à 4, dans lequel la fréquence de rotation des paires de secteurs d'aimant est inférieure ou égale à 10 Hz.

6. Appareil selon l'une quelconque des revendications 1 à 5, dans lequel la bobine de fil conducteur (14) est une bobine circulaire centrée sur l'axe de rotation des secteurs angulaires d'aimant.

7. Appareil selon l'une quelconque des revendications 1 à 6, dans lequel la bobine de fil conducteur (14) est une bobine à double anneaux.

8. Appareil selon l'une quelconque des revendications 1 à 7, dans lequel chaque secteur angulaire d'aimant comprend une même forme géométrique avec une ouverture angulaire intérieure (α) au niveau de l'axe de rotation de 90°, une ouverture angulaire extérieure (β) au niveau d'une extrémité libre opposée à l'axe de rotation comprise entre 20° et 50°, et deux bords latéraux (8-1 à 8-4) définissant un rayon s'étendant sur une distance (d) qui est comprise entre un tiers et deux-tiers d'une distance (D/2) séparant l'axe de rotation de l'extrémité libre des secteurs angulaires d'aimant.

9. Appareil selon l'une quelconque des revendications 1 à 8, comprenant en outre une couche de circuit magnétique disposée au dos des paires de secteurs angulaires d'aimants.

10. Appareil selon l'une quelconque des revendications 1 à 9, comprenant en outre une couche de blindage magnétique disposée autour des paires de secteurs angulaires d'aimant.

## Patentansprüche

1. Vorrichtung (2) zur Erzeugung eines sehr niederfrequenten gepulsten Magnetfeldes, das von einem sehr niederfrequenten Wechselmagnetfeld getragen wird und dazu ausgelegt ist, auf eine Zone des menschlichen Körpers angewendet zu werden, wobei die Vorrichtung Folgendes umfasst:
eine Vielzahl von Paaren von winkligen Magnetsektoren (6-1 bis 6-4) mit umgekehrter Polarität, wobei die Paare von winkligen Magnetsektoren auf einer gleichen Rotationsachse (X-X) zentriert sind, winklig voneinander beabstandet und mit einer vorbestimmten Geschwindigkeit um die Drehachse in Drehung versetzt sind, um ein Wechselmagnetfeld mit einer vordefinierten Frequenz zu erzeugen;
mindestens eine Spule aus leitendem Draht (14), die auf der Rotationsachse der winkligen Magnetsektoren zentriert und mit gepulstem elektrischem Strom versorgt wird, um ein gepulstes magnetisches Feld zu erzeugen, das sich über das erzeugte magnetische Wechselfeld legt, indem die winkligen Magnetsektoren in Drehung versetzt werden; und
einen unabhängigen elektrischen Motor zur elektrischen Versorgung der Spule, wobei die winkligen Magnetsektoren (6-1 bis 6-4) durch den elektrischen Motor um die Drehachse (X-X) in Drehung versetzt werden.

2. Vorrichtung nach Anspruch 1, wobei die Spule aus leitendem Draht (14) durch eine gepulste elektrische Spannung versorgt wird, die die Form von regelmäßigen Pulsierungen aufweist, die eine Dauer von weniger als 1 ms und eine Frequenz von 20 Hz aufweist und eine Intensität erzeugt, die ausreichend ist, um ein magnetisches Feld von 1 bis 2 mT zu erzeugen.

3. Vorrichtung nach Anspruch 2, umfassend außerdem Mittel, um während eines Verwendungszyklus die Frequenz der Pulsierungen des gepulsten elektrischen Stroms variieren zu lassen.

4. Vorrichtung nach einem der Ansprüche 1 bis 3, umfassend außerdem Mittel, um während eines Verwendungszyklus die Rotationsfrequenz der Paare von Magnetsektoren variieren zu lassen.

5. Vorrichtung nach einem der Ansprüche 1 bis 4, wobei die Rotationsfrequenz der Paare von Magnetsektoren kleiner als oder gleich wie 10 Hz ist.

6. Vorrichtung nach einem der Ansprüche 1 bis 5, wobei die Spule mit leitendem Draht (14) eine kreisförmige Spule ist, die auf der Rotationsachse der winkligen Magnetsektoren zentriert ist.

7. Vorrichtung nach einem der Ansprüche 1 bis 6, wobei die Spule mit leitendem Draht (14) eine Spule mit doppeltem Ring ist.

8. Vorrichtung nach einem der Ansprüche 1 bis 7, wobei jeder winklige Magnetsektor eine gleiche geometrische Form mit einer inneren winkligen Öffnung (α) auf der Ebene der Rotationsachse von 90°, eine äußere winklige Öffnung (β) auf der Ebene eines freien Endes gegenüber der Rotationsachse im Bereich zwischen 20° und 50° und zwei seitlichen Rändern (8-1 bis 8-4) umfasst, die einen Strahl definieren, der sich über eine Distanz (d) erstreckt, die im Bereich zwischen einem Drittel und zwei Drittel einer Distanz (D/2) liegt, die die Rotationsachse von dem freien Ende der winkligen Magnetsektoren trennt.

9. Vorrichtung nach einem der Ansprüche 1 bis 8, umfassend außerdem eine magnetische Schaltschicht, die auf der Rückseite der Paare von winkligen Magnetsektoren angeordnet ist.

10. Vorrichtung nach einem der Ansprüche 1 bis 9, umfassend außerdem eine magnetische Abschirmschicht, die um Paare von winkligen Magnetsektoren angeordnet ist.

## Claims

1. Device (2) for generating a very-low-frequency, pulsed magnetic field carried by a very-low-frequency alternating magnetic field, and intended to be applied to a region of the human body, the device comprising:
a plurality of pairs of angular magnet sectors (6-1 to 6-4) of opposite polarity, the pairs of angular magnet sectors being centred on one same axis of rotation (X-X), angularly spaced apart from one another, and set in rotation at a predetermined speed about the axis of rotation so as to generate an alternating magnetic field at a predefined frequency;
at least one conductive wire coil (14) centred on the axis of rotation of the angular magnet sectors and supplied with a pulsed electric current so as to generate a pulsed magnetic field superimposed over the alternating magnetic field generated by the setting in rotation of the angular magnet sectors, and
an electric motor independent of the electric supply to the coil, the angular magnet sectors (6-1 to 6-4) being driven in rotation about the axis of rotation (X-X) by said electric motor.

2. The device according to claim 1, wherein the conductive wire coil (14) is supplied with a pulsed electric voltage in the form of regular pulses of less than 1 ms duration, frequency of 20 Hz and generating sufficient intensity to generate a magnetic field of 1 to 2 mT.

3. The device according to claim 2, further comprising means to cause the pulse frequency of the pulsed electric current to vary during a cycle of use.

4. The device according to any of claims 1 to 3, further comprising means to cause the rotation frequency of the pairs of magnet sectors to vary during a cycle of use.

5. The device according to any of claims 1 to 4, wherein the rotation frequency of the pairs of magnet sectors is less than or equal to 10 Hz.

6. The device according to any of claims 1 to 5, wherein the conductive wire coil (14) is a circular coil centred on the axis of rotation of the angular magnet sectors.

7. The device according to any of claims 1 to 6, wherein the conductive wire coil (14) is a figure-of-eight coil.

8. The device according to any of claims 1 to 7, wherein each angular magnet sector has one same geometric shape with an inner angular opening (α) of 90° at the axis of rotation, an outer angular opening (β) of between 20° and 50° at a free end opposite the axis of rotation, and two side edges (8-1 to 8-4) defining a radius extending over a distance (d) of between one third and two thirds of a distance (D/2) separating the axis of rotation from the free end of the angular magnet sectors.

9. The device according to any of claims 1 to 8 further comprising a magnetic circuit layer arranged on the back of the pairs of angular magnet sectors.

10. The device according to any of claims 1 to 9 further comprising a magnetic shielding layer arranged around the pairs of angular magnet sectors.
